# NOUVEAU FASCICULE DE BREVET EUROPEEN

(11) **EP 0 966 955 B2**
(45) Date de publication et mention de la décision concernant l'opposition: **23.03.2005**
(45) Mention de la délivrance du brevet: 21.11.2001
(21) Numéro de dépôt: 99401430.6
(22) Date de dépôt: 11.06.1999
(51) Int. Cl.: A61K 7/48, A61K 7/06

(54) **Composition cosmétique contenant un polyorganosiloxane et un terpolymère acrylique et utilisation de cette composition pour le traitement des matières kératiniques**
Kosmetische Zusammensetzung enthaltend ein Polyorganosiloxan und ein Acrylterpolymer und Verwendung dieser Zusammensetzung zur Behandlung von Keratinmaterial
Cosmetic composition containing a polyorganosiloxane and an acrylic terpolymer and use of said composition for treating keratinous materials

(30) Priorité: 15.06.1998 FR 9807512
(43) Date de publication de la demande: 29.12.1999
(73) Titulaire: L'OREAL, 75008 Paris (FR)
(72) Inventeur: Dupuis, Christine, 75018 Paris (FR)
(74) Mandataire: Casalonga, Axel

(56) Documents cités:
- EP-A- 0 521 748
- EP-A- 0 824 914
- EP-A- 0 875 557
- US-A- 5 292 843
- US-A- 5 294 693
- Roempps Chemielexikon, 1997, p. 3855

## Description

La présente invention concerne des compositions cosmétiques contenant en association, au moins un polyorganosiloxane et un terpolymère acrylique ainsi que l'utilisation de ces compositions pour le traitement des matières kératiniques.

Les silicones sont des produits cosmétiques particulièrement recherchés pour leurs propriétés de conditionnement, leurs propriétés adoucissantes et démêlantes, notamment en formulation capillaire.

Il est souvent nécessaire, lorsqu'on utilise certaines silicones telles que celles insolubles en milieu aqueux, de les introduire dans un support épaissi ou gélifié pour les maintenir en suspension et obtenir une composition stable.

Il est également important de disposer de compositions non pâteuses, non grasses, et s'étalant bien sur la peau et les cheveux.

Dans le but d'obtenir un support épaissi ou gélifié, on utilise des polymères épaississants et/ou gélifiants. On connaît des polymères gélifiants comportant dans leur chaîne une partie hydrophile et une partie hydrophobe constituée d'une chaîne grasse comme le produit "PEMULEN TR1" commercialisé par la société GOODRICH ou les polymères "ACRYSOL" commercialisés par la société ROHM & HAAS. Le polymère "PEMULEN TR1" donne des formulations de texture pâteuse ne s'étalant pas bien. Le polymère "ACRYSOL" n'a pas un bon pouvoir gélifiant et donne des formulations troubles et instables.

On connaît aussi par la demande EP-A-0 824 914 des polymères épaississants ou modificateurs de rhéologie utilisables dans des compositions cosmétiques comprenant
a) un monomère acrylate ou méthacrylate,
b) un monomère hétérocyclique vinyl-substitué, méthacrylamide, alkylaminoalkyl(meth)acrylate ou alkylaminoalkyl (méth)acrylamide et éventuellement
c) un monomère associatif pouvant être, entre autres, un uréthane non-ionique provenant de la réaction d'un tensio-actif non-ionique monohydrique avec un monoisocyanate insaturé monoéthylénique.

La demanderesse a découvert de manière surprenante qu'en utilisant une famille de polymères épaississants et/ou gélifiants et en les associant à des polyorganosiloxanes, on pouvait obtenir des formulations cosmétiques présentant une viscosité satisfaisante à un pH relativement peu élevé, qui sont stables, non grasses, non pâteuses et qui s'étalent bien sur la peau et les cheveux.

La présente invention a donc pour objet des compositions cosmétiques contenant, dans un support aqueux cosmétiquement acceptable, au moins un polyorganosiloxane et un terpolymère acrylique que l'on définira plus en détail dans la suite de la description.

Ce polymère permet notamment de préparer des compositions aqueuses ou hydro-organiques contenant des solvants cosmétiquement acceptables, rincées ou non rincées, allant des produits légèrement gélifiés aux sticks ou bâtonnets solides.

Ce terpolymère présente l'avantage d'être stable en milieu électrolyte et d'avoir un très bon pouvoir épaississant à pH égal ou supérieur à 5,5 permettant d'atteindre un bon niveau de viscosité. Il permet en outre d'utiliser des concentrations élevées en alcool.

Ce polymère, utilisé en association avec au moins un polyorganosiloxane, permet de réaliser des produits gélifiés non pâteux, ayant une bonne facilité d'étalement, doux à l'application et stables à la conservation.

Il permet également d'améliorer l'effet de conditionnement des silicones sur les cheveux, notamment leur toucher, leur douceur et leur aptitude au démêlage.

Le terpolymère acrylique utilisé conformément à l'invention est soluble ou gonflable dans les alcalis. Il est caractérisé par le fait qu'il comprend
a) 20 à 70% en poids, d'un acide carboxylique à insaturation α, β-monoéthylénique ;
b) au moins 20% en poids, d'un monomère à insaturation monoéthylénique non tensio-actif différent de a) et
c) 0,5 à 60% en poids, d'un monomère uréthane non-ionique susceptible d'être obtenu par réaction d'un tensio-actif non-ionique monohydrique avec un monoisocyanate à insaturation monoéthylénique.

De préférence, le terpolymère comprend les monomères a), b) et c) à raison de 25-55, au moins 30 et respectivement au moins 10 % en poids.

L'acide carboxylique à insaturation α, β-monoéthylénique a) peut être choisi en particulier parmi l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'amino méthyl propanol ou l'amino méthyl propane diol.

Le terpolymère doit aussi contenir une proportion importante indiquée ci-dessus d'un monomère b) à insaturation monoéthylénique qui n'a pas de propriété tensio-active. Les monomères préférés sont ceux qui donnent des polymères insolubles dans l'eau lorsqu'ils sont homopolymérisés et sont illustrés par les acrylates et méthacrylates d'alkyle en C₁-C₄ comme acrylate de méthyle, l'acrylate d'éthyle, l'acrylate de butyle ou les méthacrylates correspondants. Les monomères plus particulièrement préférés sont les acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensio-actifs non-ioniques monohydriques utilisés pour obtenir le monomère uréthane non-ionique c) sont bien connus et sont généralement des composés hydrophobes alcoxylés contenant des unités d'oxyde d'alkylène formant la partie hydrophile de la molécule. Les hydrophobes sont généralement constitués par un résidu d'alcool aliphatique ou d'un alkylphénol dans lesquels une chaîne carbonée contenant au moins six atomes de carbone constitue la partie hydrophobe du tensio-actif.

Les tensio-actifs non-ioniques monohydriques préférés ont pour formule : 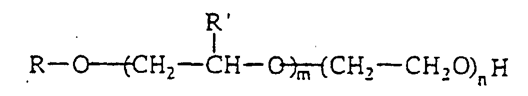 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Le monoisocyanate monoéthylénique préféré est 1'α,α-diméthyl-m-isopropényl-benzylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

A titre de terpolymères pouvant être utilisés selon l'invention, on peut citer les produits de la polymérisation en émulsion d'acide méthacrylique comme composant a), d'acrylate d'éthyle comme composant b) et d'un macromonomère uréthane non-ionique comme composant c), ayant la structure suivante : 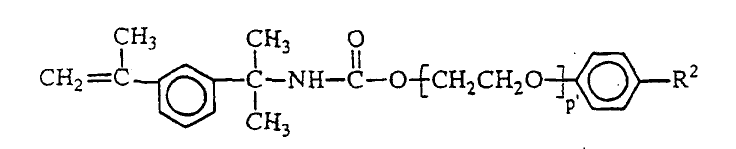 dans laquelle p' va de 6 à 150 et est égal de préférence à 30 et R² est un radical alkyle en C₈-C₁₃, tel que celui décrit dans l'exemple 3 de la demande de brevet EP-A-0 173 109.

Le terpolymère acrylique préféré utilisé selon l'invention est obtenu à partir d'acide méthacrylique comme composant a), d'acrylate de méthyle comme composant b) et d'un macromonomère uréthane non-ionique comme composant (c), ayant la structure suivante : 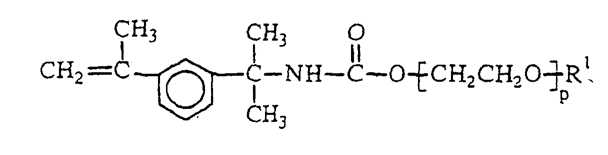 dans laquelle p va de 6 à 150 et R¹ est un radical alkyle en C₁₈-C₂₆, de préférence en C₂₀-C₂₄ linéaire, d'origine végétale, tel que le radical docosyle.

Le terpolymère acrylique est présent dans les compositions cosmétiques de l'invention dans des concentrations allant de 0,01 à 20% en poids par rapport au poids total de la composition et préférentiellement de 0,1 à 10% en poids.

Les polyorganosiloxanes modifiés ou non utilisés dans les compositions selon la présente invention sont des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes utilisés conformément à la présente invention, on peut citer à titre non limitatif :

### I. Les silicones volatiles

Celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges.

On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane.

### II. Les silicones non volatiles

Elles sont constituées principalement par:
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes organomodifiés ;
(vii) les copolymères blocs ayant un bloc linéaire polysiloxane-polyalkylène comme unité répétitive ;
(viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(x) ou leurs mélanges.

Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHONE POULENC; l'huile "47 V 500 000" de RHONE POULENC ou certaines "VISCASIL" de GENERAL ELECTRIC, ou "MIRASIL" de RHONE POULENC et les polydiméthylsiloxanes linéaires à groupements terminaux hydroxydiméthylsilyle, telles que les huiles de la série 48 V de RHONE POULENC.

Dans cette classe de polyalkylsiloxanes, on peut également mentionner les polyalkylsiloxanes vendus par la Société GOLDSCHMIDT sous les dénominations "ABILWAX 9800" et "ABILWAX 9801", qui sont des polyalkyl (C₁-C₂₀) siloxanes.

Parmi les polyalkylarylsiloxanes, on peut citer les polyméthylphénylsiloxanes, les polydiméthylméthylphénylsiloxanes, les polydiméthyldiphénylsiloxanes linéaires et ramifiés, tels que par exemple :
- l'huile "RHODORSIL 763" de RHONE POULENC,
- les huiles "SILBIONE 70641 V 30 et 70641 V 200" de RHONE POULENC,
- le produit "DC 556 Cosmetic Grad Fluid" de DOW CORNING,
- les silicones des séries PK de BAYER, telles que la "PK20",
- les silicones des séries PN, PH de BAYER, comme les "PN 1000" et "PH 1000",
- certaines huiles des séries SF de GENERAL ELECTRIC, telles que les SF 1250, SF 1265, SF 1154, SF 1023.

Les gommes de silicone, conformes à la présente invention, sont des polydiorganosiloxanes de masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane))(phénylméthylsiloxane)],
- poly[(diméthylsiloxane)/(diphénylsiloxane)/(méthylvinylsiloxane)].

On peut citer, par exemple, à titre non limitatif, les mélanges suivants :
**1**) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING :
**2**) les mélanges formés à partir d'une gomme polydiméthylsiloxane avec une silicone cyclique, tels que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
**3**) les mélanges de deux PDMS de viscosité différente, notamment d'une gomme PDMS et d'une huile PDMS, tels que les produits "SF 1236" et "CF 1241" de la Société GENERAL ELECTRIC. Le produit "SF 1236" est le mélange d'une gomme SE 30 définie ci-dessus d'une viscosité de 20 m²/s et d'une huile SF 96 d'une viscosité de 5.10⁻⁵ m²/s (15% de gomme SE 30 et 85% d'huile SF 96). Le produit "CF 1241" est le mélange d'une gomme SE 30 (33%) et d'une PDMS (67%) de viscosité 10⁻³m²/s.

Les résines de polyorganopolysiloxanes utilisables conformément à l'invention sont de préférence des systèmes siloxaniques réticulés renfermant les unités :
R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593" ou ceux vendus sous les dénominations "SILICONE FLUID SS 4230" et "SS 4267" par la Société GENERAL ELECTRIC et qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant
**a**) des groupements polyéthylèneoxy et/ou polypropylèneoxy, comportant éventuellement des groupes alkyle, telles que :
   · le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200",
   · les huiles "SILWET" L 722, L 7500, L 77, L 711 de la Société UNION CARBIDE,
   · le mélange de diméthicone copolyol et de cyclométhicone tel que le produit vendu sous la dénomination "Q2-3225C" par la Société DOW CORNING ;
   · le produit "MIRASIL DMCO" vendu par RHONE POULENC.
**b**) des groupements (per)fluorés comme les groupements trifluoroalkyle, telles que, par exemple, celles vendues par la Société GENERAL ELECTRIC sous les dénominations "FF.150 Fluorosilicone Fluid" ou par la Société SHIN ETSU sous les dénominations "X-22-819"; "X-22-820"; "X-22-821"; "X-22-822"; ou "FL 100" ;
**c**) des groupements hydroxyacylamino, telles que celles décrites dans la demande de brevet européen EP-A-0 342 834 et en particulier la silicone vendue par la Société DOW CORNING sous la dénomination "Q2-8413" ;
**d**) des groupements thiols comme dans les silicones "X 2-8360" de DOW CORNING ou les "GP 72A" et "GP 71" de GENESEE ;
**e)** des groupements aminés substitués ou non, comme dans la "GP4 SILICONE FLUID" de GENESEE, la "GP 7100" de GENESEE, la "Q2 8220" de DOW CORNING, 1"'AFL 40" d'UNION CARBIDE ou la silicone dénommée "AMODIMETHICONE" dans le dictionnaire CTFA ;
**f**) des groupements carboxylates, comme les produits décrits dans le brevet européen EP 186 507 de CHISSO CORPORATION;
**g**) des groupements hydroxylés, comme les polyorganosiloxanes à fonction hydroxyalkyle, décrits dans la demande de brevet FR-A-2 589 476, et en particulier les polyorganosiloxanes à fonction γ-hydroxypropyle ;
**h**) des groupements alcoxylés comportant au moins 12 atomes de carbone comme le produit "SILICONE COPO-LYMER F 755" de SWS SILICONES et les produits "ABILWAX 2428", "ABILWAX 2434", "ABILWAX 2440" de la Société GOLDSCHMIDT;
**i**) des groupements acyloxyalkyle comportant au moins 12 atomes de carbone, comme par exemple les polyorganosiloxanes décrits dans la demande de brevet FR-A-2 641 185, et en particulier les polyorganosiloxanes à fonction stéaroyloxypropyle.
**j**) des groupements ammonium quaternaire, comme dans les produits "X2 81 08" et "X2 81 09", le produit "ABIL K 3270" de la Société GOLDSCHMIDT ;
**k**) des groupements amphotères ou bétaïniques, tels que dans le produit vendu par la Société GOLDSCHMIDT sous la dénomination "ABIL B 9950" ;
**l**) des groupements bisulfite, tels que dans les produits vendus par la Société GOLDSCHMIDT sous les dénominations "ABIL S 201" et "ABIL S 255".

Les copolymères blocs ayant un bloc linéaire polysiloxane-polyoxyalkylène comme unité répétitive utilisés dans le cadre de la présente invention ont de préférence la formule générale suivante :

([Y(R₂SiO)ₐR'₂SiYO][CₙH₂ₙO)_{b}])_{c} (V)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100,
- b est un nombre entier supérieur ou égal à 4, de préférence compris entre 4 et 200 et encore plus particulièrement entre 5 et 100,
- c est un nombre entier supérieur ou égal à 4, de préférence compris entre 4 et 1000 et encore plus particulièrement entre 5 et 300 ;
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre 400 et 10.000, celui de chaque bloc polyoxyalkylène étant compris entre 300 et 10.000,
- les blocs siloxane représentent de 10% à 95% en poids du copolymère bloc,
- le poids moléculaire moyen du copolymère bloc étant d'au moins 3.000 et de préférence compris entre 5.000 et 1.000.000 et encore plus particulièrement entre 10.000 et 200.000.

R et R' sont préférentiellement choisis parmi le groupe comprenant les radicaux alkyle comme par exemple les radicaux méthyle, éthyle, propyle, butyle, pentyle, hexyle, octyle, décyle, dodécyle, les radicaux aryle comme par exemple phényle, naphtyle, les radicaux aralkyle comme par exemple benzyle, phényléthyle, les radicaux tolyle, xylyle, et cyclohexyle.

Y est de préférence -R"-, -R"-CO-, -R"-NHCO-, -R"-NH-CO-NH-R"-NHCO, -R"-OCONH-R"'-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄C₆H₄-, -C₆H₄-CH₂- C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical -C₄H_{8⁻}.

La préparation des copolymères blocs mis en oeuvre dans le cadre de la présente invention est décrite dans la demande européenne EP 0 492 657 A1.

Les copolymères blocs linéaires polysiloxane-polyoxyalkylène préférés selon l'invention sont choisis parmi ceux de formule :

[C₄H₈O(CₙH₂ₙO)_{b} (CₘH₂ₘO)_{d} - C₄H₈ - SiMe₂(O:SiMe₂)ₐ SiMe₂]_{c} (VI)

où Me représente méthyle, n et m sont des entiers allant de 2 à 4, a est un entier supérieur ou égal à 4, de préférence compris entre 5 et 200, b et d sont des entiers supérieurs ou égaux à 0, de préférence compris entre 4 et 200, b + d est supérieur ou égal à 4, de préférence compris entre 4 et 200 et c est un nombre supérieur ou égal à 4, de préférence compris entre 4 et 1000.

Parmi ces copolymères, on utilise plus particulièrement ceux ayant un motif récurrent de formule :

[-(SiMe₂O)ₓ SiMe₂-C₄H₈O-(C₂H₄O)_{y}-(C₃H₆O)_{z} - C₄H₈-] (VII)

où x est un nombre compris entre 5 et 15 (bornes incluses), y est un nombre compris entre 15 et 30 (bornes incluses) ; et z est un nombre compris entre 20 et 40 (bornes incluses).

Parmi ces polymères, on utilise plus particulièrement ceux dont le rapport en poids siloxane/polyoxyalkylène est d'environ 20 et le rapport en poids polyoxyéthylène/polyoxypropylène est d'environ 65/35.

On peut également choisir des polymères dont l'unité répétitive est de formule (VI) et dont le rapport en poids siloxane/polyoxyalkylène est d'environ 75 et le rapport en poids polyoxyéthylène/polyoxypropylène est d'environ 50/50, des polymères dont le rapport en poids siloxane/polyoxyalkylène est d'environ 35 et le rapport en poids polyoxyéthylène/polyoxypropylène est d'environ 100/0, des polymères dont le rapport en poids siloxane/polyoxyalkylène est d'environ 30 et le rapport en poids polyoxyéthylène/polyoxypropylène est d'environ 0/100.

Selon un mode de réalisation particulier de l'invention, le copolymère bloc est choisi parmi les copolymères suivants :

[[(CH₃)₂SiO]₄₁(CH₃)₂SiCH₂CH(CH₃)CH₂―O(C₂H₄O)₁₈-(C₃H₆O)₃₃CH₂CH(CH₃)CH₂]₁₆₋₁

[[(CH₃)₂SiO]₃₁(CH₃)₂SiCH₂CH(CH₃)CH₂―O(C₂H₄O)₂₀―(C₃H₆O)₂₉CH₂CH(CH₃)CH₂]₁₃₋₃

[[(CH₃)₂SiO]₉(CH₃)₂SiCH₂CH(CH₃)CH₂―O(C₂H₄O)₂₀―(C₃H₆O)₂₉CH₂CH(CH₃)CH₂]₂₆₋₃

[[(CH₃)₂SiO]₁₆(CH₃)₂SiCH₂CH(CH₃)CH₂―O(C₂H₄O)₁₈―(C₃H₆O)₂₀CH₂CH(CH₃)CH₂]₂₁₋₅

[[(CH₃)₂SiO]₉(CH₃)₂SiCH₂CH(CH₃)CH₂―O(C₂H₄O)₅―CH₂CH(CH₃)CH₂]₄₋₈

Les polymères à squelette organique non-siliconé greffé par des monomères contenant un polysiloxane, conformes à l'invention, sont choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Des exemples de polymères à squelette polysiloxanique greffé par des monomères organiques non siliconés convenant à la mise en oeuvre de la présente invention, ainsi que leur mode particulier de préparation, sont notamment décrits dans les demandes de brevets EP-A-0 582 152, WO 93/23009 et WO 95/03776.

Les polyorganosiloxanes préférés pour être utilisés selon l'invention sont les polyorganopolysiloxanes non volatils.

Les polyorganosiloxanes sont utilisés dans les compositions de l'invention dans des proportions comprises entre 0,01 et 50% en poids et de préférence entre 0,1 et 30% en poids par rapport au poids total de la composition.

Les compositions selon l'invention contiennent un milieu aqueux cosmétiquement acceptable. Elles présentent un pH pouvant aller de 3,5 à 11, de préférence compris entre 5,5 et 11, et encore plus préférentiellement entre 5,5 et 8,5.

Le milieu cosmétiquement acceptable des compositions selon l'invention est plus particulièrement constitué d'eau et éventuellement de solvants organiques cosmétiquement acceptables.

Les solvants organiques peuvent représenter de 0,5 à 90% du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les mono-alcools inférieurs linéaires ou ramifiés ayant de 1 à 8 atomes de carbone, les polyéthylène glycols ayant de 6 à 80 unités d'oxyde d'éthylène et les polyols.

Comme solvants organiques amphiphiles, on peut citer les dérivés de polypropylène glycol (PPG) comme les esters de polypropylène glycol et d'acide gras tels que le PPG-36 oléate ou encore les éthers de PPG et d'alcool gras comme le PPG-23 oléyl éther.

Comme solvants organiques lipophiles, on peut citer par exemple les esters gras tels que l'adipate de diisopropyle, l'adipate de dioctyle, les benzoates d'alkyle, le malate de dioctyle.

Afin que les compositions cosmétiques de l'invention soient plus agréables à utiliser (plus douces à l'application, plus nourrissantes, plus émollientes), il est possible d'ajouter une phase grasse dans le milieu de ces compositions.

La phase grasse peut représenter jusqu'à 50% du poids total de la composition.

Cette phase grasse peut comporter une huile ou une cire ou leurs mélanges, et peut comprendre également des acides gras, des alcools gras et des esters d'acides gras. Les huiles peuvent être choisies parmi les huiles animales, végétales, minérales ou de synthèse et notamment parmi l'huile de vaseline, l'huile de paraffine, les isoparaffines, les poly-α-oléfines, les huiles fluorées et perfluorées. De même, les cires peuvent être choisies parmi les cires animales, fossiles, végétales, minérales ou de synthèse connues en soi.

Les compositions de l'invention peuvent contenir des adjuvants habituels dans le domaine cosmétique comme d'autres gélifiants et/ou épaississants classiques; des émulsionnants; des tensio-actifs; des agents hydratants ; des émollients ; des filtres solaires ; des actifs hydrophiles ou lipophiles comme des céramides ; des agents anti-radicaux libres ; des séquestrants ; des antioxydants ; des conservateurs ; des agents alcalinisants ou acidifiants ; des parfums ; des charges ; des matières colorantes, des réducteurs. Les quantités de ces différents adjuvants sont celles classiquement utilisées dans les domaines considérés.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion, sous forme de gels aqueux ou hydroalcooliques, sous forme de dispersions vésiculaires, sous forme d'émulsions simples ou complexes ((H/E, E/H, H/E/H ou E/H/E) et être de consistance liquide, semi-liquide ou solide telles que des laits, des crèmes, des gels, des gels-crèmes, des pâtes, des sticks et éventuellement être conditionnées en aérosol et se présenter sous la forme de mousses ou de sprays. Ces compositions sont préparées selon les méthodes usuelles.

Les compositions selon l'invention peuvent être utilisées comme produits capillaires rincés ou non-rincés, notamment pour le lavage, la coloration, le soin, le conditionnement, le défrisage, le maintien de la coiffure ou la mise en forme permanente ou non des cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray ou une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être également utilisées comme produits de soin ou d'hygiène tels que des crèmes de protection, de traitement ou de soin pour le visage, pour les mains ou pour le corps, des laits corporels de protection ou de soin, des lotions, gels ou mousses pour le soin ou le nettoyage de la peau.

Les compositions de l'invention peuvent être également utilisées comme compositions antisolaires.

Les compositions peuvent également consister en des préparations solides constituant des savons ou des pains de nettoyage.

Les compositions de l'invention peuvent être également utilisées comme produits de soin bucco-dentaire tels que des pâtes dentifrices, des bains de bouche.

Les compositions peuvent être des produits pour le maquillage tels que des crèmes pour le visage, des fonds de teint, des mascaras, des eye-liners, des rouges à lèvres, des vernis à ongles.

Un autre objet de l'invention est un procédé de traitement non-thérapeutique cosmétique de la peau, du cuir cheveu, des cheveux, des cils, des sourcils, des ongles ou des muqueuses, caractérisé par le fait qu'on applique sur le support kératinique une composition telle que définie ci-dessus, selon la technique d'utilisation habituelle de cette composition, par exemple application de crèmes, de gels, de sérums, de lotions, de laits sur la peau, le cuir chevelu ou les muqueuses.

Les exemples suivants illustrent l'invention sans présenter un caractère limitatif.

### EXEMPLE 1 : Shampooing

- Lauryl éther sulfate de sodium à 2,2 moles d'oxyde d'éthylène 17 g MA
- Cocoylbétaïne 2,8 g MA
- Terpolymère acide méthacrylique/méthyl acrylate/uréthane dérivé de diméthyl-méta-isopropényl-benzyl-isocyanate et d'alcool béhénylique éthoxylé (40 OE) en dispersion aqueuse à 25% 1 g MA
- Polydiméthylsiloxane de masse moléculaire 250.000 et de viscosité 0,5 m²/s vendu sous la dénomination "MIRASIL DM 500.000" par RHONE POULENC 2,5 g - Eau qsp 100 g
pH ajusté à 6,5 (NaOH)
MA signifie "matière active".

Ce shampooing se présente sous l'aspect d'un liquide épaissi et stable après une semaine à température ambiante. Il possède de bonnes propriétés moussantes et laisse les cheveux doux après le shampooing.

### EXEMPLE COMPARATIF 2 : Shampooing

- Lauryl éther sulfate de sodium à 2,2 moles d'oxyde d'éthylène 17 g MA
- Cocoylbétaïne 2,8 g MA
- Polyuréthane à terminaison alkyle polyéthoxylé en solution à 35% dans un mélange propylène glycol/eau (60/40) ("ACRYSOL 44" vendu par la société ROHM & HAAS) 1 g MA
- Polydiméthylsiloxane de masse moléculaire 250.000 et de viscosité 0,5 m²/s vendu sous la dénomination "MIRASIL DM 500.000" par RHONE POULENC 2.5 g
- Eau qsp 100 g
pH ajusté à 6,5 (NaOH)

Ce shampooing se présente sous l'aspect d'un liquide trouble épaissi instable, qui décante au bout d'une semaine à température ambiante.

### EXEMPLE 3 : Soin capillaire gélifié non-rincé

- Terpolymère acide méthacrylique/méthyl acrylate/uréthane dérivé de diméthyl-méta-isopropényl-benzyl-isocyanate et d'alcool béhénylique éthoxylé (40 OE) en dispersion aqueuse à 25% 1 g MA
- Polydiméthylsiloxane de masse moléculaire 250.000 et de viscosité 0,5 m²/s vendu sous la dénomination "MIRASIL DM 500.000" par RHONE POULENC 1 g
- 2-amino 2-méthyl propanol-1 (AMP) pH ajusté à 7.5 qs
- Parfum, conservateur, colorant q.s
- Eau déminéralisée qsp 100 g

On obtient un gel stable, épais, non pâteux et s'étalant bien sur les cheveux. Ce gel donne aux cheveux un toucher doux et une bonne aptitude au démêlage et possède de bonnes propriétés de fixation.

Si on remplace le terpolymère ci-dessus par le polyuréthane "ACRYSOL 44" utilisé dans l'exemple comparatif 2, on obtient un liquide épaissi, non gélifié, trouble et instable.

Si on remplace le terpolymère par le copolymère acide acrylique/acrylate d'alkyle en C₁₀/C₃₀ réticulé "PE-MULEN TR1" vendu par GOODRICH, on obtient un gel pâteux, ne s'étalant pas très bien et conférant aux cheveux des caractéristiques médiocres de douceur et démêlage.

### EXEMPLE 4 : Soin capillaire gélifié non-rincé

- Terpolymère acide méthacrylique/méthyl acrylate/uréthane dérivé de diméthyl-méta-isopropényl-benzyl-isocyanate et d'alcool béhénylique éthoxylé (40 OE) en dispersion aqueuse à 25% 1 g MA
- Polydiméthylsiloxane de viscosité 5 x 10⁻⁵ m²/s vendu sous la dénomination "MIRASIL DM 50" par RHONE POULENC 1 g
- 2-amino 2-méthyl propanol-1 (AMP) pH ajusté à 7.5 qs
- Parfum, conservateur, colorant q.s
- Eau déminéralisée qsp 100 g

On obtient un gel stable, épais, non pâteux et s'étalant bien sur les cheveux. Ce gel donne aux cheveux un toucher doux et une bonne aptitude au démêlage et possède de bonnes propriétés fixantes.

Si on remplace le terpolymère ci-dessus par le polyuréthane "ACRYSOL 44" utilisé dans l'exemple comparatif 2, on obtient un liquide épaissi, non gélifié, trouble et instable.

Si on remplace le terpolymère par le copolymère acide acrylique/acrylate d'alkyle en C₁₀/C₃₀ réticulé "PE-MULEN TR1" vendu par GOODRICH, on obtient un gel pâteux, ne s'étalant pas très bien et conférant aux cheveux des caractéristiques médiocres de douceur ; il possède en outre de mauvaises propriétés fixantes.

### EXEMPLE 5: Soin capillaire gélifié non-rincé

- Terpolymère acide méthacrylique/méthyl acrylate/uréthane dérivé de diméthyl-méta-isopropényl-benzyl-isocyanate et d'alcool béhénylique éthoxylé (40 OE) en dispersion aqueuse à 25% 1 g MA
- Polydiméthylsiloxane oxyéthyléné et oxypropyléné, de viscosité 1,5 ± 0,2 x 10⁻³ m²/s vendu sous la dénomination "MIRASIL DMCO" par RHONE POULENC 1 g
- 2-amino 2-méthyl propanol-1 (AMP) pH ajusté à 7.5 qs
- Parfum, conservateur, colorant q.s
- Eau déminéralisée qsp 100 g

On obtient un gel stable, épais, non pâteux et s'étalant bien sur les cheveux. Ce gel donne aux cheveux un toucher doux et une bonne aptitude au démêlage et possède de bonnes propriétés fixantes.

Si on remplace le terpolymère ci-dessus par le polyuréthane "ACRYSOL 44" utilisé dans l'exemple comparatif 2, on obtient un liquide épaissi, non gélifié, trouble et instable.

Si on remplace le terpolymère par le copolymère acide acrylique/acrylate d'alkyle en C₁₀/C₃₀ réticulé "PE-MULEN TR1" vendu par GOODRICH, on obtient un gel pâteux, ne s'étalant pas très bien et conférant aux cheveux des caractéristiques très médiocres de douceur et démêlage ; il possède en outre de mauvaises propriétés fixantes.

### EXEMPLE 6 : Gel-crème solaire haute protection

- 4 tert.butyl 4'-méthoxy dibenzoylméthane ("PARSOL 1789" vendu par la société ROCHE) 2 g
- Acide benzène 1,4-di(3-méthylidène-10-camphosulfonique) en solution aqueuse à 33% 1,5 g
- 2-cyano-3,3-diphénylacrylate de 2-éthylhexyle ("UVINUL N 539" vendu par la société BASF) 10 g
- Terpolymère acide méthacrylique/méthyl acrylate/uréthane dérivé de diméthyl-méta-isopropényl-benryl-isocyanate et d'alcool béhénylique éthoxylé (40 OE) en dispersion aqueuse à 25% 3 g MA
- Polydiméthyl/méthylsiloxane oxyéthyléné et oxypropyléné, à 10% dans D4/D5 1 g MA
- Cyclohexadiméthylsiloxane 5 g
- Isohexadécane 10 g
- Stéarate d'oligomère d'acide 12-hydroxystéarique 0,5 g
- Hydratants 8 g
- Séquestrant q.s.
- Triéthanolamine q.s. pH 7
- Eau déminéralisée stérilisée qsp 100 g

On obtient un gel-crème stable, homogène et s'étalant bien sur la peau.

## Revendications

1. Composition cosmétique destinée au traitement des matières kératiniques, **caractérisée par le fait qu'**elle comprend, dans un support aqueux cosmétiquement acceptable, au moins un polyorganosiloxane et un terpolymère acrylique comprenant :
a) 20 à 70% en poids, d'un acide carboxylique à insaturation α, β-monoéthylénique ;
b) au moins 20% en poids, d'un monomère à insaturation monoéthylénique non tensio-actif différent de a) et
c) 0,5 à 60% en poids d'un monomère uréthane non-ionique susceptible d'être obtenu par réaction d'un tensio-actif non-ionique monohydrique avec un monoisocyanate à insaturation monoéthylénique.

2. Composition selon la revendication 1, **caractérisée par le fait que** l'acide carboxylique à insaturation α, β-monoéthylénique a) est choisi parmi l'acide acrylique, l'acide méthacrylique, l'acide itaconique et l'acide maléique.

3. Composition selon la revendication 2, **caractérisée par le fait que** l'acide carboxylique à insaturation α, β-monoéthylénique a) est l'acide méthacrylique.

4. Composition selon l'une quelconque des revendications 1 à 3, **caractérisée par le fait que** le monomère à insaturation monoéthylénique non tensio-actif b) est choisi parmi les acrylates et méthacrylates d'alkyle en C₁-C₄, le styrène, le vinyltoluène, l'acétate de vinyle, l'acrylonitrile et le chlorure de vinylidène.

5. Composition selon la revendication 4, **caractérisée par le fait que** le monomère à insaturation monoéthylénique non tensio-actif est l'acrylate de méthyle ou d'éthyle.

6. Composition selon l'une quelconque des revendications 1 à 5, **caractérisée par le, fait que** le tensio-actif non-ionique monohydrique susceptible d'être utilisé pour obtenir le monomère uréthane non-ionique c) a pour formule : 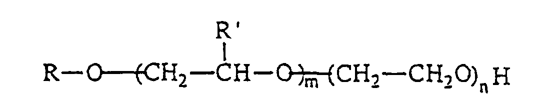 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

7. Composition selon la revendication 6, **caractérisée par le fait que** R est choisi parmi les radicaux dodécyle, alkyle en C₁₈-C₂₆ et alkyl (C₈-C₁₃) phényle, m = 0 et n est un nombre moyen allant d'environ 5 à 150.

8. Composition selon l'une quelconque des revendications 1 à 7, **caractérisée par le fait que** le monoisocyanate à insaturation monoéthylénique susceptible d'être utilisé pour former le monomère uréthane non-ionique c) est 1'α, α-diméthyl m-isopropényl benzyl isocyanate.

9. Composition selon l'une quelconque des revendications 1 à 8, **caractérisée par le fait que** le terpolymère acrylique est susceptible d'être obtenu en dispersion aqueuse à partir d'acide méthacrylique comme composant a), d'acrylate de méthyle comme composant b) et d'un macromonomère uréthane non-ionique de structure suivante : 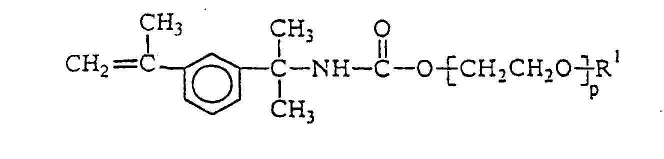 dans laquelle p va de 6 à 150 et R¹ est un radical alkyle en C₁₈-C₂₆, comme composant c).

10. Composition selon l'une quelconque des revendications 1 à 9, **caractérisée par le fait que** le terpolymère acrylique est présent dans des concentrations allant de 0,01 à 20% en poids, par rapport au poids total de la composition.

11. Composition selon l'une quelconque des revendications 1 à 10, **caractérisée par le fait que** les polyorganosiloxanes sont choisis parmi les silicones volatiles cycliques contenant 3 à 7 atomes de silicium et ayant un point d'ébullition compris entre 60°C et 260°C.

12. Composition selon l'une quelconque des revendications 1 à 10, **caractérisée par le fait que** les polyorganosiloxanes sont des silicones non volatiles choisies dans le groupe constitué par :
(i) les polyalkylsiloxanes ;
(ii) les polyarylsiloxanes ;
(iii) les polyalkylarylsiloxanes ;
(iv) les gommes de silicone ;
(v) les résines de silicone ; ..
(vi) les polyorganosiloxanes organomodifiés, comportant dans leur structure générale, un ou plusieurs groupements organofonctionnels directement fixés sur la chaîne siloxanique ou fixés par l'intermédiaire d'un radical hydrocarboné ;
(vii) les copolymères blocs ayant un bloc linéaire polysiloxane-polyalkylène comme unité répétitive ;
(viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(x) ou leurs mélanges.

13. Composition selon la revendication 12, **caractérisée par le fait que** les polyalkylsiloxanes sont choisis dans le groupe constitué par :
- les polydiméthylsiloxanes linéaires à groupements terminaux triméthylsilyle, et
- les polydiméthylsiloxanes linéaires à groupements terminaux hydroxydiméthylsilyle.

14. Composition selon la revendication 12, **caractérisée par le fait que** les gommes de silicone sont des polydiorganosiloxanes de masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges.

15. Composition selon la revendication 14, **caractérisée par le fait que** les gommes de silicone sont choisies parmi les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane))(phénylméthylsiloxane)],
- poly[(diméthylsiloxane)/(diphénylsiloxane)/(méthylvinylsiloxane)].

16. Composition selon la revendication 14, **caractérisée par le fait que** les gommes de silicone sont choisies dans le groupe constitué par :
a) les mélanges formés à partir d'un polydiméthylsiloxane hydroxylé en bout de chaîne et d'un polydiméthylsiloxane cyclique ;
b) les mélanges formés à partir d'une gomme de polydiméthylsiloxane avec une silicone cyclique ;
c) les mélanges de deux polydiméthylsiloxanes de viscosité différente.

17. Composition selon la revendication 12, **caractérisée par le fait que** les résines de polyorganosiloxane sont des systèmes siloxaniques réticulés réticulés renfermant les unités : R₂SiO_{2/2}, RSiO_{3/2} et SiO_{4/2} dans lesquelles R représente un groupement hydrocarboné possédant 1 à 6 atomes de carbone ou un groupement phényle.

18. Composition selon la revendication 12, **caractérisée par le fait que** les polyorganosiloxanes organomodifiés sont choisis parmi ceux comportant
a) des groupements polyéthylèneoxy et/ou polypropylèneoxy, comprtant éventuellement des groupes alkyle ;
b) des groupements (per)fluorés ;
c) des groupements hydroxyacylamino ;
d) des groupements thiols ;
e) des groupements carboxylates ;
f) des groupements hydroxylés :
h) des groupements alcoxylés
i) des groupements acyloxyalkyle ;
j) des groupements aminés substitués ou non ;
k) des groupements ammonium quaternaire ;
l) des groupements amphotères ou bétaïniques ;
m) des groupements bisulfites.

19. Composition selon la revendication 12, **caractérisée par le fait que** le copolymère bloc linéaire polysiloxane-polyoxyalkylène répond à la formule générale :
([Y(R₂SiO)ₐR'₂SiYO] [CₙH₂ₙO)_{b}])_{c} (V)
dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentant de 10% environ à 95% environ en poids du copolymère bloc,
- le poids moléculaire moyen du copolymère bloc étant d'au moins 3.000.

20. Composition selon l'une quelconque des revendications 1 à 19, **caractérisée par le fait que** le ou les polyorganosiloxanes sont présents dans des concentrations allant de 0,01 à 50% en poids, par rapport au poids total de la composition.

21. Composition selon l'une quelconque des revendications 1 à 20, **caractérisée par le fait qu'**elle présente un pH allant de 3,5 à 11.

22. Composition selon l'une quelconque des revendications 1 à 21, **caractérisée par le fait que** le milieu aqueux cosmétiquement acceptable est constitué d'eau ou d'eau et au moins un solvant organique choisi dans le groupe constitué par les solvants organiques hydrophiles, lipophiles, amphiphiles ou leurs mélanges.

23. Composition selon l'une quelconque des revendications 1 à 22, **caractérisée par le fait qu'**elle comprend en outre au moins un adjuvant cosmétique usuel choisi parmi les corps gras, les gélifiants et/ou épaississants classiques, les tensio-actifs, les agents hydratants, les émollients, les filtres solaires, les actifs hydrophiles ou lipophiles comme les céramides, les agents anti-radicaux libres, les séquestrants, les antioxydants, les conservateurs, les agents alcalinisants ou acidifiants, les parfums, les charges, les matières colorantes et les réducteurs.

24. Composition selon l'une quelconque des revendications 1 à 23, **caractérisée par le fait qu'**elle se présente sous forme d'émulsion, de lotion, de gel, de dispersion vésiculaire, de pâte, de bâtonnet solide ou est conditionnée en aérosol et se présente sous forme de mousse ou de spray.

25. Composition selon l'une quelconque des revendications 1 à 24, **caractérisée par le fait qu'**elle constitue un produit capillaire rincé ou non rincé pour le lavage, la teinture, le soin, le conditionnement,le défrisage, le maintien de la coiffure ou la mise en forme permanente ou non des cheveux, une composition anti-solaire, un produit de soin bucco-dentaire ou un produit de maquillage.

26. Composition selon l'une quelconque des revendications 1 à 25 **caractérisée par le fait que** dans le macromonomère uréthane-non-ionique c), R¹ est un radical alkyle en C₂₀-C₂₄ linéaire, d'origine végétale, tel que le radical docosyle.

27. Composition selon l'une quelconque des revendications 1 à 26 **caractérisée par le fait que** le terpolymère acrylique est présent dans des concentrations allant de 0,1 à 10% en poids par rapport au poids total de la composition.

28. Composition selon l'une quelconque des revendications 1 à 27 **caractérisée par le fait qu'**elle présente un pH allant de 5,5 à 8,5.

29. Procédé de traitement non-thérapeutique cosmétique pour la protection de la peau, du cuir chevelu, des cheveux, des cils, des sourcils, des ongles et des muqueuses, **caractérisé par le fait qu**'on applique sur ces derniers une quantité efficace d'une composition telle que définie selon l'une quelconque des revendications 1 à 28.

## Patentansprüche

1. Kosmetische Zusammensetzung zur Behandlung von Keratinsubstanzen,
**dadurch gekennzeichnet, daß**
sie in einem kosmetisch akzeptablen Träger mindestens ein Polyorganosiloxan und ein Acrylterpolymer enthält, welches aufweist:
a) 20 bis 70 Gew.-% einer Carbonsäure mit α,β-monoethylenischer Doppelbindung,
b) mindestens 20 Gew.-% eines Monomers mit monoethylenischer Doppelbindung, das kein grenzflächenaktiver Stoff und von a) verschieden ist, und
c) 0,5 bis 60 Gew.-% eines nichtionischen Urethanmonomers, das durch Umsetzung eines nichtionischen grenzflächenaktiven Stoffes mit einer Hydroxygruppe mit einem Monoisocyanat mit monoethylenischer Doppelbindung hergestellt werden kann.

2. Zusammensetzung nach Anspruch 1, **dadurch gekennzeichnet; daß** die Carbonsäure mit α,β-monoethylenischer Doppelbindung a) unter Acrylsäure, Methacrylsäure, Itaconsäure und Maleinsäure ausgewählt ist.

3. Zusammensetzung nach Anspruch 2, **dadurch gekennzeichnet, daß** die Carbonsäure mit α,β-monoethylenischer Doppelbindung a) die Methacrylsäure ist.

4. Zusammensetzung nach einem der Ansprüche 1 bis 3, **dadurch gekennzeichnet, daß** das Monomer mit monoethylenischer Doppelbindung b), das kein grenzflächenaktiver Stoff ist, unter den C₁₋₄-Alkylacrylaten und C₁₋₄-Alkylmethacrylaten, Styrol, Vinyltoluol, Vinylacetat, Acrylnitril und Vinylidenchlorid ausgewählt ist.

5. Zusammensetzung nach Anspruch 4, **dadurch gekennzeichnet, daß** das Monomer mit monoethylenischer Doppelbindung, das kein grenzflächenaktiver Stoff ist, das Methylacrylat oder Ethylacrylat ist.

6. Zusammensetzung nach einem der Ansprüche bis 5, **dadurch gekennzeichnet, daß** der nichtionische grenzflächenaktive Stoff mit einer Hydroxygruppe, der zur Herstellung des nichtionischen Urethanmonomers c) verwendet werden kann, die folgende Formel aufweist: 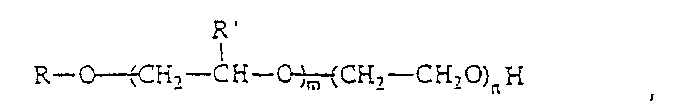 worin R eine C₆₋₃₀-Alkylgruppe oder C₈₋₃₀-Aralkylgruppe bedeutet, R' eine C₁₋₄-Alkylgruppe ist, n einen Mittelwert von 5 bis 150 und m einen Mittelwert von 0 bis 50 bedeutet, mit der Maßgabe, daß n mindestens so groß wie m ist und n + m = 5 - 150.

7. Zusammensetzung nach Anspruch 6, **dadurch gekennzeichnet, daß** R unter den Gruppen Dodecyl, C₁₈₋₂₆-Alkyl und C₈₋₁₃-Alkylphenyl ausgewählt ist, m = 0 und n einen Mittelwert von etwa 5 bis 150 bedeutet.

8. Zusammensetzung nach einem der Ansprüche 1 bis 7, **dadurch gekennzeichnet, daß** das Monoisocyanat mit monoethylenischer Doppelbindung, das zur Bildung des nichtionischen Urethanmonomers c) verwendet werden kann, das α,α-Dimethyl-m-isopropylbenzylisocyanat ist.

9. Zusammensetzung nach einem der Ansprüche 1 bis 8, **dadurch gekennzeichnet, daß** das Acrylterpolymer in wäßriger Dispersion ausgehend von Methacrylsäure als Komponente a), Methylacrylat als Komponente b) und einem nichtionischen Urethanmakromer der folgenden Formel als Komponente c): 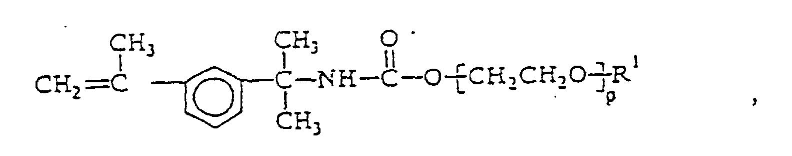 worin p im Bereich von 6 bis 150 liegt und R¹ eine C₁₈₋₂₆-Alkylgruppe bedeutet, hergestellt werden kann.

10. Zusammensetzung nach einem der Ansprüche 1 bis 9, **dadurch gekennzeichnet, daß** das Acrylterpolymer in Konzentrationen von 0,01 bis 20 Gew.-%, bezogen auf das Gesamtgewicht der Zusammensetzung, vorliegt.

11. Zusammensetzung nach einem der Ansprüche 1 bis 10, **dadurch gekennzeichnet, daß** die Polyorganosiloxane unter den cyclischen flüchtigen Siliconen mit 3 bis 7 Siliciumatomen und einem Siedepunkt von 60 bis 260 °C ausgewählt sind.

12. Zusammensetzung nach einem der Ansprüche 1 bis 10, **dadurch gekennzeichnet, daß** die Polyorganosiloxane nicht flüchtige Silicone sind, die ausgewählt sind unter:
(i) Polyalkylsiloxanen;
(ii) Polyarylsiloxanen;
(iii) Polyalkylarylsiloxanen;
(iv) Silicongummis;
(v) Siliconharzen;
(vi) organomodifizierten Polyorganosiloxanen, die in ihrer allgemeinen Struktur eine oder mehrere direkt an die Sitoxangruppe gebundene oder über eine Kohlenwasserstoffgruppe an die Siloxangruppe gebundene organofunktionelle Gruppen aufweisen;
(vii) Blockcopolymere, die als wiederkehrende Einheit einen geraden Polysiloxan-Polyalkylen-Block enthalten;
(viii) gepfropfte Siliconpolymere mit einem nicht siliconhaltigen organischen Grundgerüst, die aus einer organischen Hauptkette bestehen, die aus organischen Monomeren gebildete wird, die kein Silicon enthalten, auf die im Inneren der Kette sowie gegebenenfalls an mindestens einem ihrer Enden mindestens ein Polysiloxanmakromer gepfropft ist;
(ix) gepfropften Siliconpolymeren mit Polysiloxangrundgerüst, das mit nicht siliconhaltigen organischen Monomeren gepfropft ist, die eine Polysiloxanhauptkette enthalten, auf die im Inneren der Kette sowie gegebenenfalls an mindestens einem ihrer Enden mindestens ein organisches Makromergepfropft ist, das kein Silicon enthält;
(x) oder deren Gemischen.

13. Zusammensetzung nach Anspruch 12, **dadurch gekennzeichnet, daß** die Polyalkylsiloxane ausgewählt sind unter:
- geradkettigen Polydimethylsiloxanen mit Trimethylsilylendgruppen und
- geradkettigen Polydimethylsiloxanen mit Hydroxydimethylsilylendgruppen.

14. Zusammensetzung nach Anspruch 12, **dadurch gekennzeichnet, daß** die Silicongummis Polydiorganosiloxane mit einem Molekulargewicht von 200 000 bis 1 000 000 sind, die einzeln oder im Gemisch in einem Lösungsmittel verwendet werden, das unter den flüchtigen Siliconen, Polydimethylsiloxanölen (PDMS), Polyphenylmethylsiloxanölen (PPMS), Isoparaffinen, Methylenchlorid, Pentan, Dodecan, Tridecan und Tetradecan oder deren Gemischen ausgewählt ist.

15. Zusammensetzung nach Anspruch 14, **dadurch gekennzeichnet, daß** die Silicongummis unter den folgenden Verbindungen ausgewählt sind:
- Polydimethylsiloxan,
- Poly[(dimethylsiloxan)/(methylvinylsiloxan)],
- Poly[(dimethylsiloxan)/(diphenylsiloxan)],
- Poly[(dimethylsiloxan)/(phenylmethylsiloxan)],
- Poly[(dimethylsiloxan)/(diphenylsiloxan)/(methylvinylsiloxan)].

16. Zusammensetzung nach Anspruch 14, **dadurch gekennzeichnet, daß** die Silicongummis ausgewählt sind unter:
a) Gemischen eines Polydimethylsiloxans, das am Kettenende hydroxyliert ist, und eines cyclischen Dimethylsiloxans;
b) Gemischen eines Polydimethylsiloxangummis und eines cyclischen Silicons;
c) Gemischen von zwei Polydimethylsiloxanen unterschiedlicher Viskosität.

17. Zusammensetzung nach Anspruch 12, **dadurch gekennzeichnet, daß** die Polyorganosiloxanharze vernetzte Siloxansysteme sind, die folgende Einheiten enthalten: R₂SiO_{2/2}, RSiO_{3/2} und SiO_{4/2}, worin R eine Kohlenwasserstoffgruppe mit 1 bis 6 Kohlenstoffatomen oder eine Phenylgruppe bedeutet.

18. Zusammensetzung nach Anspruch 12, **dadurch gekennzeichnet, daß** die organomodifizierten Polyorganosiloxane unter den Verbindungen ausgewählt sind, die enthalten:
a) Polyethylenoxy- und/oder Polypropylenoxygruppen, die gegebenenfalls Alkylgruppen enthalten,
b) (per)fluorierte Gruppen,
c) Hydroxyacylaminogruppen,
d) Thiolgruppen,
e) Carboxylatgruppen,
f) hydroxylierte Gruppen,
h) alkoxylierte Gruppen,
i) Acyloxyalkygruppen,
j) substituierte oder unsubstituierte aminierte Gruppen,
k) quartäre Ammoniumgruppen,
I) amphotere Gruppen oder Betaingruppen,
m) Bisulfitgruppen.

19. Zusammensetzung nach Anspruch 12, **dadurch gekennzeichnet, daß** das lineare Polysiloxan-Polyoxyalkylen-Blockcopolymer der allgemeinen Formel entspricht:
([Y(R₂SiO)aR'₂SiYO][CₙH₂ₙO)_{b}])_{c} (V),
worin bedeuten:
- R und R', die identisch oder voneinander verschieden sind, eine einwertige Kohlenwasserstoffgruppe, die keine aliphatische Doppelbindung aufweist,
- n eine ganze Zahl von 2 bis 4,
- a 5 oder eine ganze Zahl über 5,
- b 4 oder eine ganze Zahl über 4,
- c 4 oder eine ganze Zahl über 4,
- Y eine zweiwertige organische Gruppe, die über eine Kohlenstoff-Silicium-Bindung an das angrenzende Siliciumatom und über ein Sauerstoffatom an einen Polyoxyalkylenblock gebunden ist,
- wobei das mittlere Molekulargewicht jedes Siloxanblocks im Bereich von etwa 400 bis etwa 10 000 und jedes Polyoxyalkylenblocks im Bereich von etwa 300 bis etwa 10 000 liegt,
- wobei die Siloxanblöcke etwa 10 bis etwa 95 Gew.-% des Blockcopolymers ausmachen und
- wobei das mittlere Molekulargewicht des Blockcopolymers mindestens 3000 beträgt.

20. Zusammensetzung nach einem der Ansprüche 1 bis 19, **dadurch gekennzeichnet, daß** das Polyorganosiloxan oder die Polyorganosiloxane in Konzentrationen von 0,01 bis 50 Gew.-%, bezogen auf das Gesamtgewicht der Zusammensetzung, vorliegen.

21. Zusammensetzung nach einem der Ansprüche 1 bis 20, **dadurch gekennzeichnet, daß** sie einen pH-Wert von 3,5 bis 11 aufweist.

22. Zusammensetzung nach einem der Ansprüche 1 bis 21, **dadurch gekennzeichnet, daß** das kosmetisch akzeptable wäßrige Medium aus Wasser oder Wasser und mindestens einem organischen Lösungsmittel besteht, das unter den hydrophilen, lipophilen oder amphiphilen organischen Lösungsmitteln oder deren Gemischen ausgewählt ist.

23. Zusammensetzung nach einem der Ansprüche 1 bis 22, **dadurch gekennzeichnet, daß** sie ferner mindestens einen üblichen kosmetischen Zusatzstoff enthält, der unter den Fettsubstanzen, herkömmlichen Gelbildnern und/oder Verdickungsmitteln, grenzflächenaktiven Stoffen, Hydratisierungsmitteln, Emollentien, Sonnenschutzfiltern, hydrophilen oder lipophilen Wirkstoffen wie Ceramiden, Mitteln gegen freie Radikale, Maskierungsmitteln, Antioxidantien, Konservierungsmitteln, Alkalisierungsmitteln oder Ansäuerungsmittelnn, Parfums, Füllstoffen, Farbmitteln und Reduktionsmitteln ausgewählt ist.

24. Zusammensetzung nach einem der Ansprüche 1 bis 23, **dadurch gekennzeichnet, daß** sie als Emulsion, Lotion, Gel, Vesikeldispersion, Paste oder fester Stift vorliegt oder als Aerosol konfektioniert ist und in Form von Schaum oder Spray vorliegt.

25. Zusammensetzung nach einem der Ansprüche 1 bis 24, **dadurch gekennzeichnet, daß** sie ein Produkt für das Haar, das ausgespült wird oder im Haar verbleibt, zum Waschen, Färben, zur Pflege, zur Konditionierung, zum Entkräuseln, für den Halt der Frisur oder die Formgebung der Haare, die dauerhaft odervorübergehend sein kann, eine Zusammensetzung zum Sonnenschutz, ein Produkt zur Mund- und Zahnpflege oder ein Produkt zum Schminken darstellt.

26. Zusammensetzung nach einem der Ansprüche 1 bis 25, **dadurch gekennzeichnet, daß** in dem nichtionischen Urethanmakromer c) R¹ eine geradkettige C₂₀₋₂₄-Alkylgruppe pflanzlichen Ursprungs, wie Docosyl, bedeutet.

27. Zusammensetzung nach einem der Ansprüche 1 bis 26, **dadurch gekennzeichnet, daß** das Acrylterpolymer in Konzentration von 0,1 bis 10 Gew.-%, bezogen auf das Gesamtgewicht der Zusammensetzung, vorliegt.

28. Zusammensetzung nach einem der Ansprüche 1 bis 27, **dadurch gekennzeichnet, daß** sie einen pH-Wert von 5,5 bis 8,5 aufweist.

29. Verfahren zur nicht therapeutischen kosmetischen Behandlung zum Schutz der Haut, der Kopfhaut, der Haare, der Wimpern, der Augenbrauen, der Nägel und der Schleimhäute, **dadurch gekennzeichnet, daß** auf diese eine Zusammensetzung nach einem der Ansprüche 1 bis 28 in einer wirksamen Menge aufgebracht wird.

## Claims

1. Cosmetic composition intended for treating keratinous material, **characterized in that it** comprises, in a cosmetically acceptable aqueous support, at least one polyorganosiloxane and an acrylic terpolymer comprising:
a) 20 to 70% by weight of a carboxylic acid containing a, β-monoethylenic unsaturation;
b) at least 20% by weight of a non-surfactant monomer containing monoethylenic unsaturation, which is different from a), and
c) 0.5 to 60% by weight of a nonionic urethane monomer which can be obtained by reacting a monohydric nonionic surfactant with a monoisocyanate containing monoethylenic unsaturation.

2. Composition according to Claim 1, **characterized in that** the carboxylic acid containing α,β-monoethylenic unsaturation a) is chosen from acrylic acid, methacrylic acid, itaconic acid and maleic acid.

3. Composition according to Claim 2, **characterized in that** the carboxylic acid containing α,β-monoethylenic unsaturation a) is methacrylic acid.

4. Composition according to any one of Claims 1 to 3, **characterized in that** the non-surfactant monomer containing monoethylenic unsaturation b) is chosen from C₁-C₄ alkyl acrylates and methacrylates, styrene, vinyltoluene, vinyl acetate, acrylonitrile and vinylidene chloride.

5. Composition according to Claim 4, **characterized in that** the non-surfactant monomer containing monoethylenic unsaturation is methyl or ethyl acrylate.

6. Composition according to any one of Claims 1 to 5, **characterized in that** the monohydric nonionic surfactant which can be used to obtain the nonionic urethane monomer c) has the formula: 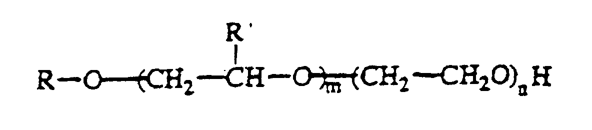 in which R is a C₆-C₃₀ alkyl or C₈-C₃₀ aralkyl group, R' is a C₁-C₄ alkyl group, n is an average number ranging from 5 to 150 and m is an average number ranging from 0 to 50, with the condition that n is at least as large as m and that n+m = 5-150.

7. Composition according to Claim 6, **characterized in that** R is chosen from dodecyl, C₁₈-C₂₆ alkyl and (C₈-C₁₃) alkylphenyl radicals, m = 0 and n is an average number ranging approximately from 5 to 150.

8. Composition according to any one of Claims 1 to 7, **characterized in that** the monoisocyanate containing monoethylenic unsaturation which can be used to form the nonionic urethane monomer c) is α,α-dimethyl-m-isopropenyl-benzyl isocyanate.

9. Composition according to any one of Claims 1 to 8, **characterized in that** the acrylic terpolymer can be obtained as an aqueous dispersion from methacrylic acid as component a), from methyl acrylate as component b) and from a nonionic urethane macromonomer of the following structure: 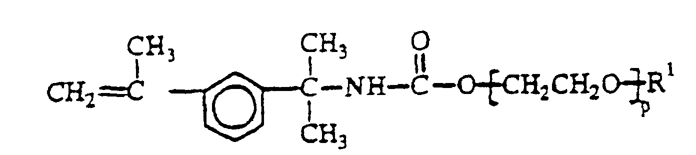 in which p ranges from 6 to 150 and R¹ is a C₁₈-C₂₆ alkyl radical, as component c).

10. Composition according to any one of Claims 1 to 9, **characterized in that** the acrylic terpolymer is present in concentrations ranging from 0.01 to 20% by weight relative to the total weight of the composition.

11. Composition according to any one of Claims 1 to 10, **characterized in that** the polyorganosiloxanes are chosen from cyclic volatile silicones containing 3 to 7 silicon atoms and having a boiling point of between 60°C and 260°C.

12. Composition according to any one of Claims 1 to 10, **characterized in that** the polyorganosiloxanes are nonvolatile silicones chosen from the group consisting of:
(i) polyalkylsiloxanes;
(ii) polyarylsiloxanes;
(iii) polyalkylarylsiloxanes;
(iv) silicone gums;
(v) silicone resins;
(vi) organomodified polyorganosiloxanes containing, in their general structure, one or more organofunctional groups directly attached to the siloxane chain or attached via a hydrocarbon-based radical;
(vii) block copolymers having a polysiloxane-polyalkylene linear block as repeating unit;
(viii) grafted silicone polymers containing a non-silicone organic skeleton, consisting of an organic main chain formed from organic monomers not containing silicone, onto which is grafted, within the said chain as well as, optionally, on at least one of its ends, at least one polysiloxane macromonomer;
(ix) grafted silicone polymers containing a polysiloxane skeleton, grafted with non-silicone organic monomers, comprising a polysiloxane main chain onto which is grafted, within the said chain as well as, optionally, on at least one of its ends, at least one organic macromonomer containing no silicone;
(x) or mixtures thereof.

13. Composition according to Claim 12,
**characterized in that** the polyalkylsiloxanes are chosen from the group consisting of:
- linear polydimethylsiloxanes containing trimethylsilyl end groups, and
- linear polydimethylsiloxanes containing hydroxydimethylsilyl end groups.

14. Composition according to Claim 12,
**characterized in that** the silicone gums are polydiorganosiloxanes with a molecular mass of between 200,000 and 1,000,000, used alone or as a mixture in a solvent chosen from volatile silicones, polydimethylsiloxane (PDMS) oils, polyphenylmethylsiloxane (PPMS) oils, isoparaffins, methylene chloride, pentane, dodecane, tridecane and tetradecane, or mixtures thereof.

15. Composition according to Claim 14,
**characterized in that** the silicone gums are chosen from the following compounds:
- polydimethylsiloxane;
- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methylvinylsiloxane)].

16. Composition according to Claim 14, **characterized in that** the silicone gums are chosen from the group consisting of
a) mixtures formed from a polydimethylsiloxane which is hydroxylated at the end of the chain and from a cyclic polydimethylsiloxane;
b) mixtures formed from a polydimethylsiloxane gum with a cyclic silicone;
c) mixtures of two polydimethylsiloxanes of different viscosity.

17. Composition according to Claim 12, **characterized in that** the polyorganosiloxane resins are crosslinked siloxane systems containing the units: R₂SiO_{2/2}, RSiO_{3/2} and SiO_{4/2} in which R represents a hydrocarbon-based group having 1 to 6 carbon atoms or a phenyl group.

18. Composition according to Claim 12,
**characterized in that** the organomodified polyorganosiloxanes are chosen from those containing:
a) polyethyleneoxy and/or polypropyleneoxy groups, optionally containing alkyl groups;
b) (per)fluoro groups;
c) hydroxyacylamino groups;
d) thiol groups;
e) carboxylate groups;
f) hydroxylated groups;
h) alkoxylated groups;
i) acyloxyalkyl groups;
j) substituted or unsubstituted amine groups;
k) quaternary ammonium groups;
l) amphoteric or betaine groups;
m) bisulphite groups.

19. Composition according to Claim 12, **characterized in that** the polysiloxane-polyoxyalkylene linearblock copolymer corresponds to the general formula:
([Y(R₂SiO)ₐR'₂SiYO][CₙH₂ₙO)_{b}])_{c} (V)
in which
- R and R', which may be identical or different, represent a monovalent hydrocarbon-based radical containing no aliphatic unsaturation,
- n is an integer ranging from 2 to 4,
- a is an integer greater than or equal to 5,
- b is an integer greater than or equal to 4,
- c is an integer greater than or equal to 4,
- Y represents a divalent organic group which is linked to the adjacent silicon atom via a carbon-silicon bond and to a polyoxyalkylene block via an oxygen atom,
- the average molecular weight of each siloxane block is between about 400 and about 10,000, that of each polyoxyalkylene block being between about 300 and about 10,000,
- the siloxane blocks representing from about 10% to about 95% of the weight of the block copolymer,
- the average molecular weight of the block copolymer being at least 3000.

20. Composition according to any one of Claims 1 to 19, **characterized in that** the polyorganosiloxane(s) is(are) present in concentrations ranging from 0.01 to 50% by weight relative to the total weight of the composition.

21. Composition according to any one of Claims 1 to 20, **characterized in that** it has a pH ranging from 3.5 to 11.

22. Composition according to any one of Claims 1 to 21, **characterized in that** the cosmetically acceptable aqueous medium consists of water or of water and at least one organic solvent chosen from the group consisting of hydrophilic, lipophilic and amphiphilic organic solvents, or mixtures thereof.

23. Composition according to any one of Claims 1 to 22, **characterized in that** it also comprises at least one common cosmetic adjuvant chosen from fatty substances, standard gelling agents and/or thickeners, surfactants, moisturizers, emollients, sunscreens, hydrophilic or lipophilic active agents such as ceramides, anti-free-radical agents, sequestering agents, antioxidants, preserving agents, basifying or acidifying agents, fragrances, fillers, dyestuffs and reducing agents.

24. Composition according to any one of Claims 1 to 23, **characterized in that** it is in the form of an emulsion, a lotion, a gel, a vesicle dispersion, a paste or a solid tube or is packaged as an aerosol and is in the form of a mousse or a spray.

25. Composition according to any one of Claims 1 to 24, **characterized in that** it constitutes a rinse-out or leave-in hair product to wash, dye, care for, condition, straighten or maintain the hairstyle or to permanently or temporarily reshape the hair, an antisun product, an oral care product or a make-up product.

26. Composition according to any one of Claims 1 to 25 **characterized in that,** in the nonionic urethane macromonomer c), R₁ is a linear C₂₀-C₂₄ alkyl radical of plant origin, such as the docosyl radical.

27. Composition according to any one of Claims 1 to 26, **characterized in that** the acrylic terpolymer is present in concentrations ranging from 0.1% to 10% by weight relative to the total weight of the composition.

28. Composition according to any one of Claims 1 to 27, **characterized in that** it has a pH ranging from 5.5 to 8.5.

29. Cosmetic non-therapeutic treatment process for protecting the skin, the scalp, the hair, the eyelashes, the eye-brows, the nails and mucous membranes, **characterized in that** an effective amount of a composition as defined according to any one of Claims 1 to 28 is applied to one of the above materials.
